# EUROPÄISCHE PATENTSCHRIFT

(11) **EP 1 900 330 B1**
(45) Veröffentlichungstag und Bekanntmachung des Hinweises auf die Patenterteilung: **07.12.2011**
(21) Anmeldenummer: 07116062.6
(22) Anmeldetag: 11.09.2007
(51) Int. Cl.: A61B 17/06, A61B 17/00

(54) **Chirurgische Nadel und Verfahren zum Herstellen einer chirurgischen Nadel**
Surgical needle and method for manufacturing a surgical needle
Aiguille chirurgicale et méthode de fabrication d'une aiguille chirurgicale

(30) Priorität: 13.09.2006 DE 102006044788; 18.09.2006 DE 202006014897 U
(43) Veröffentlichungstag der Anmeldung: 19.03.2008
(73) Patentinhaber: Aesculap AG, 78532 Tuttlingen (DE)
(72) Erfinder: Wäschle, Tobias, 78598 Königsheim (DE)
(74) Vertreter: Hoeger, Stellrecht & Partner Patentanwälte

(56) Entgegenhaltungen:
- EP-A- 0 898 939
- DE-A1- 10 259 642
- US-A1- 2003 114 882
- US-A1- 2006 047 309

## Beschreibung

Die vorliegende Erfindung betrifft eine chirurgische Nadel mit einem langgestreckten Nadelkörper und einer an einem distalen Ende des Nadelkörpers ausgebildeten Nadelspitze, wobei der Nadelkörper insbesondere aus einem langgestreckten zylindrischen Rohling hergestellt ist, wobei der Nadelkörper mindestens eine Strukturprofilseitenfläche aufweist, die mindestens einen mit einem Strukturprofil versehenen Strukturprofilseitenflächenbereich umfasst, und wobei sich der mindestens eine Strukturprofilseitenflächenbereich bis zur Nadelspitze erstreckt.

Ferner betrifft die vorliegende Erfindung ein Verfahren zum Herstellen einer chirurgischen Nadel mit einem langgestreckten Nadelkörper und einer an einem distalen Ende des Nadelkörpers ausgebildeten Nadelspitze, wobei der Nadelkörper insbesondere aus einem langgestreckten zylindrischen Rohling hergestellt wird, wobei der Nadelkörper mit mindestens einer Strukturprofilseitenfläche versehen wird, wobei die mindestens eine Strukturprofilseitenfläche zur Ausbildung mindestens eines Strukturprofilseitenflächenbereichs mit einem Strukturprofil derart versehen wird, dass sich der mindestens eine Strukturprofilseitenflächenbereich bis zur Nadelspitze erstreckt.

Chirurgische Nadeln der eingangs beschriebenen Art werden beispielsweise in Form von Mikronadeln bei chirurgischen Eingriffen am Herzen eingesetzt. Es ist bekannt, derartige Mikronadeln aus einem langgestreckten zylindrischen Rohling herzustellen, wobei der Rohling durch Pressen von vier Seiten zu einem langgestreckten quaderförmigen Nadelkörper geformt wird. Eine derartige Mikronadel weist einen im Wesentlichen quadratischen Querschnitt sowie abgerundete Längskanten auf und ungefähr einen Durchmesser von etwa 0,2 mm. Aufgrund ihrer Abmessungen ist die Stabilität solcher Mikronadeln begrenzt. Ferner ist auch das Gleitverhalten der Nadel beim Durchstechen von Körper und Gewebe begrenzt.

Chirurgische Nadeln der eingangs beschriebenen Art sind beispielsweise aus der DE 102 59 642 A1, der EP 0 898 939 A1 sowie der US 2006/0047309 A1 bekannt. Mit einer Silikonbeschichtung versehene chirurgische Nadeln sind insbesondere aus der US 2003/0114882 A1 bekannt.

Es ist daher Aufgabe der vorliegenden Erfindung, eine chirurgische Nadel, insbesondere eine Mikronadel, sowie ein Verfahren zum Herstellen einer chirurgischen Nadel so weiterzubilden, dass die Handhabbarkeit und die Herstellung der chirurgischen Nadel verbessert werden.

Diese Aufgabe wird bei einer chirurgischen Nadel der eingangs beschriebenen Art erfindungsgemäß dadurch gelöst, dass das Strukturprofil in Form eines eingeprägten Profils ausgebildet ist und eine Mehrzahl von eingeprägten Vertiefungen aufweist.

Die erfindungsgemäß vorgeschlagene Nadel hat, insbesondere in Form einer Mikronadel, den Vorteil, dass durch Ausbilden des Strukturprofilseitenflächenbereichs die Stabilität der Nadel deutlich erhöht wird. Dies betrifft zum einen eine Biegesteifigkeit der Nadel, zum anderen deren Gleitverhalten beim Durchstechen von Gewebe. Das Gleitverhalten ist im Vergleich zu bekannten chirurgischen Nadeln insbesondere auch deshalb verbessert, weil sich der Strukturprofilseitenflächenbereich bis zur Nadelspitze erstreckt, das heißt bis zum distalen Ende der Nadelspitze hin. So wird bereits beim Eindringen der Nadelspitze in Gewebe ein deutlich verbessertes Gleitverhalten erreicht. Ferner weist die erfindungsgemäße chirurgische Nadel ein verändertes Spitzendesign aufgrund des sich bis zum Ende der Spitze hin erstreckenden Strukturprofilseitenflächenbereichs auf, was ihr zudem eine einzigartige Optik verleiht. Durch die mindestens eine Strukturprofil-seitenfläche ergibt sich zudem ein besserer Sitz in einem Nadelhalter, mit dem die chirurgische Nadel bei chirurgischen Eingriffen üblicherweise gehalten wird. Insbesondere bei chirurgischen Mikronadeln ist das Strukturprofil vorzugsweise in Form eines Mikroprofils ausgebildet, und zwar mit Abmessungen der Vertiefungen, die vorzugsweise kleiner oder sogar deutlich kleiner sind als ein Durchmesser des Nadelkörpers. Die Vertiefungen können insbesondere regelmäßig angeordnet sein und das Strukturprofil einen hohen Symmetriegrad aufweisen, beispielsweise eine drei-, vier-, fünf- oder sechszählige Symmetrie.

Günstig ist es, wenn der mindestens eine Strukturprofilseitenflächenbereich mindestens die Hälfte der mindestens einen Strukturprofilseitenfläche bildet. Vorzugsweise erstreckt er sich über die gesamte Strukturprofilseitenfläche. Je größer der Strukturprofilseitenflächenbereich ist, um so besser das Gleitverhalten des Nadelkörpers beim Eindringen in Gewebe.

Das Gleitverhalten kann weiter verbessert werden, wenn der Nadelkörper zwei Strukturprofilseitenflächen aufweist und wenn die zwei Strukturprofilseitenflächen in entgegengesetzten Richtungen voneinander weg weisen. Außerdem kann der Nadelkörper so auf einfache Weise hergestellt werden, beispielsweise durch Pressen zwischen zwei aufeinander zu ausgerichteten Bearbeitungswerkzeugen in Form von Profilstempeln.

Vorteilhafterweise weist der Nadelkörper im Querschnitt eine rechteckige oder quadratische oder im Wesentlichen eine rechteckige oder quadratische Form auf. Eine solche Form lässt sich auf einfache Weise herstellen, beispielsweise durch Pressen von vier Seiten.

Günstig ist es, wenn der Nadelkörper vier ebene oder im Wesentlichen ebene Seitenflächen aufweist und wenn eine der Seitenflächen eine Strukturprofilseitenfläche ist. Ebene Seitenflächen lassen sich insbesondere auf einfache Weise durch Pressen herstellen.

Grundsätzlich wäre es denkbar, dass alle Seitenflächen des Nadelkörpers in Form von Strukturprofilseitenflächen ausgebildet sind. Günstig ist es jedoch, wenn der Nadelkörper mindestens eine an die mindestens eine Strukturprofilseitenfläche angrenzende unprofilierte Seitenfläche aufweist. Eine solche Seitenfläche ist wesentlich einfacher herzustellen als eine Strukturprofilseitenfläche.

Es wäre zwar grundsätzlich denkbar, die unprofilierte Seitenflächen eben auszubilden, jedoch ist es vorteilhaft, wenn die mindestens eine unprofilierte Seitenfläche vom Nadelkörper weg weisend konvex gekrümmt ist. Zur Ausbildung einer derartigen unprofilierten Seitenfläche ist je nach verwendetem Rohling kein weiterer Verfahrensschritt zur Herstellung der Nadel erforderlich. Vorzugsweise sind zwei unprofilierte oder im Wesentlichen unprofilierte Seitenflächen vorgesehen. So kann die Herstellung der Nadel deutlich vereinfacht werden.

Der Aufbau der Nadel lässt sich weiter vereinfachen, wenn die zwei unprofilierten oder im Wesentlichen unprofilierten Seitenflächen in entgegengesetzten Richtungen voneinander weg weisen.

Sowohl der konstruktive Aufbau als auch die Herstellung der Nadel werden besonders einfach, wenn der Nadelkörper zwei Strukturprofilseitenflächen aufweist und wenn zwei die Strukturprofilseitenflächen miteinander verbindende unprofilierte oder im Wesentlichen unprofilierte Seitenflächen vorgesehen sind.

Es wäre zwar grundsätzlich denkbar, die chirurgische Nadel geradlinig, das heißt ungekrümmt, auszubilden, es ist jedoch günstig, wenn der Nadelkörper gebogen ist. Dies verbessert insbesondere die Handhabbarkeit bei mikrochirurgischen Eingriffen deutlich.

Grundsätzlich wäre es denkbar, die Strukturprofilseitenfläche mit einem erhabenen Strukturprofil zu versehen. Gemäß einer bevorzugten Ausführungsform der Erfindung kann jedoch vorgesehen sein, dass die mindestens eine Strukturprofilseitenfläche eine äußere Oberfläche definiert und dass sich die Mehrzahl von Vertiefungen von der äußeren Oberfläche in den Nadelkörper hinein erstreckt. Dies bedeutet, dass das Strukturprofil in Form eines eingeprägten Profils ausgebildet sein kann. Ein solches Strukturprofil lässt sich beispielsweise mittels erhabene Strukturprofile aufweisender Stempel herstellen.

Um die Stabilität der Nadelspitze zu erhöhen, ist es günstig, wenn eine Tiefe der Vertiefungen bezogen auf die äußere Oberfläche der mindestens einen Strukturprofilseitenfläche ausgehend von einem proximalen Ende des mindestens einen Strukturprofilseitenflächenbereichs in Richtung auf die Nadelspitze hin abnimmt. So kann trotz verbesserten Gleitverhaltens sowohl der Nadelspitze als auch der gesamten Nadel die Stabilität der Nadel, insbesondere deren Biegesteifigkeit, trotz des ausgebildeten Strukturprofils erhöht werden.

Vorteilhafterweise ist die Mehrzahl von Vertiefungen ganz oder teilweise pyramidenförmig ausgebildet. Insbesondere können die pyramidenförmigen Vertiefungen derart ausgebildet sein, dass sich ein Strukturprofil ergibt, welches einer äußeren Struktur eines Golfballs entspricht oder zumindest ähnelt.

Die Herstellung der Nadel vereinfacht sich, wenn die Mehrzahl von Vertiefungen ganz oder teilweise in Form quadratischer Pyramiden ausgebildet ist. Selbstverständlich wäre es auch denkbar, hohlkugelige Vertiefungen auszubilden, insbesondere in Form von halben Hohlkugeln. Ferner kann vorgesehen sein, dass Kanten im Übergangsbereich der Vertiefungen zur äußeren Oberfläche der Strukturprofilseitenfläche abgerundet sind.

Besonders einfach herzustellen ist die chirurgische Nadel, wenn die Mehrzahl von Vertiefungen eingepresst ist.

Günstig ist es, wenn der Nadelkörper durch Spritzgießen hergestellt ist. Auf diese Weise lässt sich die Nadel aus unterschiedlichsten Materialien herstellen. Gemäß einer bevorzugten Ausführungsform der Erfindung kann vorgesehen sein, dass der Nadelkörper durch Metallspritzgießen (MIM - Metal Injection Molding), Pulverspritzgießen (PIM - Powder Injection Molding) oder durch Kunststoffspritzgießen hergestellt ist. Durch die beschriebenen Herstellungsweisen kann der Nadelkörper hergestellt werden, ohne dass dieser gepresst werden muss. Der Nadelkörper kann so insbesondere in einem Herstellungsschritt gefertigt werden. Insbesondere ist es nicht zwingend erforderlich, die Nadel aus einem langgestreckten zylindrischen Rohling herzustellen, so dass es sich bei diesem Merkmal um ein optionales Merkmal handelt.
Vorzugsweise ist die Nadel aus einem Metall, einer Keramik oder aus Kunststoff hergestellt. Denkbar wäre es auch, Kombinationen der genannten Materialien zur Herstellung der Nadel einzusetzen.

Herkömmliche Nadelspitzen werden vorzugsweise durch mechanische Verfahren hergestellt, beispielsweise durch Formschleifen des distalen Endes des Nadelkörpers zu einer Spitze. Um jedoch das Strukturprofil bis zum distalen Ende der auszubildenden Spitze zu erhalten, ist es vorteilhaft, wenn die Nadelspitze durch elektrochemisches Polieren hergestellt ist. Beim elektrochemischen Polieren findet ein Materialabtrag statt, allerdings wird auch in den Vertiefungen Material abgetragen, so dass diese ebenfalls teilweise abgetragen oder umgeformt werden. So ist es möglich, das im Nadelkörper ausgebildete Strukturprofil bis zum distalen Ende der Spitze zu erhalten. Je nach Ausgestaltung der Spitze kann die Tiefe der einzelnen Vertiefungen bezogen auf die äußere Oberfläche in Richtung auf das distale Ende der Spitze hin abnehmen.
Beim Einsatz chirurgischer Nadeln ist am proximalen Ende der Nadel immer ein Faden angeordnet. Grundsätzlich ist es denkbar, den Faden in einer Fadenaufnahme klemmend zu halten oder durch Kleben mit dem proximalen Ende zu verbinden. Um Fäden beliebig auswählen zu können, ist es vorteilhaft, wenn die Nadel an einem proximalen Ende mindestens ein Nadelöhr aufweist. In einer Kerbe oder einem Sackloch kann ein Faden klemmend gehalten werden. Zur individuellen Auswahl eines Fadens eignet sich insbesondere ein Nadelöhr, in das der ausgewählte Faden jederzeit eingefädelt werden kann. Nadelöhre und Sacklöcher eignen sich vorzugsweise bei Makronadeln. Jedoch können auch bei Mikronadeln Nadelöhre vorgesehen werden, wobei es bei Mikronadeln auch günstig ist, Sacklöcher oder Kerben zur Aufnahme eines Fadens vorzusehen.

Vorzugsweise ist das Nadelöhr in Form einer im Querschnitt kreisförmigen, ovalen oder langlochartigen Durchbrechung des Nadelkörpers ausgebildet. So lassen sich auf einfache Weise Fäden unterschiedlicher Querschnitte durch das Nadelöhr einfädeln.

Gemäß einer bevorzugten Ausführungsform der Erfindung kann ferner vorgesehen sein, dass die Nadelspitze und/oder der gesamte Nadelkörper ganz oder teilweise mit einer Silikonschicht überzogen sind. Durch eine Silikonbeschichtung lässt sich das Gleitverhalten der Nadel weiter verbessern. Des Weiteren haben die Vertiefungen des Strukturprofils den Vorteil, dass diese quasi als "Silikonlager" dienen können, so dass das Gleitverhalten auch bei mehreren Durchstichen von Gewebe sich nicht verschlechtert.

Die eingangs gestellte Aufgabe wird bei einem Verfahren der eingangs beschriebenen Art erfindungsgemäß dadurch gelöst, dass das Strukturprofil in Form eines eingeprägten Profils ausgebildet wird, welches eine Mehrzahl von eingeprägten Vertiefungen aufweist.

Das erfindungsgemäß vorgeschlagene Verfahren ermöglicht die Herstellung einer Nadel, insbesondere einer chirurgischen Mikronadel, deren Gleitverhalten im Vergleich zu herkömmlichen Nadeln deutlich verbessert ist und die zudem eine größere Stabilität, insbesondere eine größere Biegesteifigkeit aufweist.

Ferner eignen sich die Vertiefungen, insbesondere dann, wenn sie vollständig voneinander getrennt sind, als Reservoire für Gleit- oder Schmiermittel, beispielsweise in Form von Silikon, wenn der gesamte Nadelkörper mit einer Silikonschicht überzogen wird. So lässt sich das Gleitverhalten der Nadel insgesamt auch bei mehreren Durchstichen durch Gewebe nacheinander erhalten.

Vorzugsweise wird der mindestens eine Strukturprofilseitenflächenbereich derart ausgebildet, dass er mindestens die Hälfte der mindestens einen Strukturprofilseitenfläche bildet. Der Strukturprofilseitenflächenbereich kann jedoch auch derart ausgebildet werden, dass er die gesamte Strukturprofilseitenfläche bildet.

Je größer der Strukturprofilseitenflächenbereich ist, um so besser das Gleitverhalten und die Stabilität der Nadel.

Auf einfache Weise, beispielsweise durch Pressen, lässt sich die Nadel herstellen, wenn der Nadelkörper mit zwei Strukturprofilseitenflächen versehen wird, so dass die zwei Strukturprofilseitenflächen in entgegengesetzten Richtungen voneinander weg weisen. Eine solche Nadel hat zudem auch den Vorteil, dass sie beim Eindringen in Gewebe dieses durch die beiden profilierten Seitenflächen, auf denen das Gewebe aufgleitet, besonders leicht teilen kann.

Besonders einfach herzustellen wird die Nadel, wenn der Nadelkörper derart ausgebildet wird, dass er im Querschnitt eine rechteckige oder quadratische oder im Wesentlichen rechteckige oder quadratische Form aufweist.

Formwerkzeuge zur Herstellung der Nadel sind besonders einfach ausgeformt und kostengünstig, wenn der Nadelkörper derart ausgebildet wird, dass er vier ebene oder im Wesentlichen ebene Seitenflächen aufweist und wenn mindestens eine der Seitenflächen eine Strukturprofilseitenfläche ist.

Die Herstellung der Nadel vereinfacht sich weiter, wenn der Nadelkörper derart ausgebildet wird, dass er mindestens eine an die mindestens eine Strukturprofilseitenfläche angrenzende unprofilierte Seitenfläche aufweist.

Günstig ist es, wenn die mindestens eine unprofilierte Seitenfläche vom Nadelkörper weg weisend konvex gekrümmt ausgebildet wird. Dies ermöglicht es, zur Herstellung des Nadelkörpers insbesondere einen langgestreckten zylindrischen Rohling zu verwenden, dessen verbleibende Seitenflächen nach dem Versehen der mindestens einen Strukturprofilseitenfläche mit dem Strukturprofil nicht mehr bearbeitet werden müssen.

Vorzugsweise werden zwei unprofilierte oder im Wesentlichen unprofilierte Seitenflächen ausgebildet, die beispielsweise in einem einzigen Verfahrensschritt des Nadelkörpers durch Pressen zwischen zwei Stempeln hergestellt werden können.

Der zuletzt beschriebene optionale Verfahrensschritt wird besonders einfach, wenn die zwei unprofilierten oder im Wesentlichen unprofilierten Seitenflächen in entgegengesetzten Richtungen voneinander weg weisend ausgebildet werden.Die Stabilität der Nadel erhöht sich und die Herstellung vereinfacht sich, wenn der Nadelkörper derart ausgebildet wird, dass er zwei Strukturprofilseitenflächen aufweist und dass zwei die Strukturprofilseitenflächen miteinander verbindende unprofilierte oder im Wesentlichen unprofilierte Seitenflächen ausgebildet werden. So kann insbesondere ein im Querschnitt quadratisches oder rechteckiges Nadelprofil auf einfache Weise, beispielsweise durch zwei Formpressschritte, ausgebildet werden.

Vorzugsweise wird der Nadelkörper nach der Ausbildung des mindestens einen Strukturprofilseitenflächenbereichs gebogen. Er kann dabei insbesondere so gebogen werden, dass die Strukturprofilseitenfläche nach dem Biegeschritt weiterhin in einer Ebene liegt oder aber auch derart, dass die Strukturprofilseitenfläche aus der ursprünglich von ihr definierten Ebene heraus gekrümmt ist. Eine Krümmung der Nadel hat insbesondere den Vorteil, dass so auf einfache Weise auch Gefäße miteinander vernäht werden können.

Gemäß einer bevorzugten Variante des erfindungsgemäßen Verfahrens kann vorgesehen sein, dass die mindestens eine Strukturprofilseitenfläche eine äu-ßere Oberfläche definiert und dass die Mehrzahl von Vertiefungen von der äu-ßeren Oberfläche in den Nadelkörper hinein geformt werden. So lässt sich insbesondere ein Strukturprofil durch Einpressen mittels eines ein erhabenes Strukturprofil aufweisenden Stempels ausbilden.

Um die Stabilität der Nadel nicht zu verringern, ist es günstig, wenn eine Tiefe der Vertiefungen bezogen auf die äußere Oberfläche der mindestens einen Strukturprofilseitenfläche ausgehend von einem proximalen Ende des mindestens einen Strukturprofilseitenflächenbereichs in Richtung auf die Nadelspitze hin abnehmend ausgebildet wird. Auch wenn die Vertiefungen im Bereich des distalen Endes der Nadelspitze nicht mehr so tief sind wie Vertiefungen im Bereich des nicht zugespitzten Nadelkörpers, so wird doch das Gleitverhalten der Nadel durch die Profilierung der Nadelspitze selbst deutlich verbessert. Insbesondere lässt sich eine derart profilierte Nadelspitze durch elektrochemisches Polieren auf einfache Weise herstellen.

Besonders einfach lässt sich das Strukturprofil erzeugen, wenn die Mehrzahl von Vertiefungen ganz oder teilweise pyramidenförmig ausgebildet wird. Beispielsweise kann ein solches Strukturprofil mit einem Pressstempel erzeugt werden, welcher ein Profil erhabener Pyramiden aufweist. Insbesondere lässt sich so der Nadelkörper mit einem Strukturprofil versehen, welches die Form eines bei Golfbällen bekannten Strukturprofils aufweist.

Grundsätzlich wäre es denkbar, die Nadel durch Spritzgießen zu formen. Besonders vorteilhaft ist es jedoch, wenn die Mehrzahl von Vertiefungen eingepresst wird. Dadurch lässt sich eine Biegesteifigkeit der Nadel deutlich verbessern, und zwar aufgrund der durch das Pressen hervorgerufenen Kaltverfestigung des Nadelkörpermaterials.

Wie bereits erwähnt, kann es auch vorteilhaft sein, wenn der Nadelkörper durch Spritzgießen hergestellt wird. So kann insbesondere die Nadel in einem einzigen Arbeitsschritt hergestellt werden. Insbesondere ist es hierfür nicht erforderlich, einen Rohling bereitzustellen. Dies bedeutet, dass zur Herstellung der Nadel kein langgestreckter zylindrischer Rohling erforderlich ist. Vielmehr kann das zur Herstellung der Nadel beziehungsweise des Nadelkörpers verwendete Material auch in eine Form eingespritzt werden, die die Form der fertigen Nadel vorgibt. Das Merkmal, dass der Nadelkörper aus einem langgestreckten zylindrischen Rohling hergestellt ist, ist somit ein optionales Merkmal und nicht erfindungswesentlich.

Bei einer bevorzugten Variante des erfindungsgemäßen Verfahrens kann vorgesehen sein, dass der Nadelkörper durch Metallspritzgießen (MIM - Metal Injection Molding), Pulverspritzgießen (PIM - Powder Injection Molding) oder durch Kunststoffspritzgießen hergestellt wird. Für diese Herstellungsverfahren ist kein langgestreckter zylindrischer Rohling zur Ausbildung der Nadel erforderlich.

Je nach Einsatzzweck und gewünschter Stabilität ist es vorteilhaft, wenn die Nadel aus einem Metall, aus Kunststoff oder in Form einer Keramik durch Sintern hergestellt wird.

Grundsätzlich wäre es zwar denkbar, die Nadelspitze durch Abschleifen des Nadelkörpers auszubilden. Vorzugsweise wird die Nadelspitze jedoch durch elektrochemisches Polieren hergestellt. Insbesondere dann, wenn der Grundkörper mit einem Strukturprofil versehen wurde, kann so das Strukturprofil bis zum distalen Ende der Nadelspitze erhalten werden, da beim elektrochemischen Polieren ein Materialabtrag nicht nur an einer äußeren Oberfläche, sondern auch an Oberflächen der ausgebildeten Vertiefungen erfolgt. Dies führt zwar dazu, dass eine Tiefe der Vertiefungen bezogen auf eine äußere Oberfläche in Richtung auf das distale Ende der Nadelspitze mit zunehmender Polierdauer abnimmt, jedoch bleibt trotzdem ein Strukturprofil erhalten.

Grundsätzlich ist es möglich, einen Faden direkt an einem proximalen Ende des Nadelkörpers klemmend oder klebend zu fixieren. Vorzugsweise wird die Nadel jedoch an einem proximalen Ende mit mindestens einem Nadelöhr, einem Sackloch oder einer Kerbe versehen. Insbesondere ein Nadelöhr ermöglicht es, die Nadel mit jeweils für den gewünschten Einsatzzweck optimalem Nahtgut auszustatten. Sacklöcher und Kerben zur Fixierung des Fadens haben insbesondere den Vorteil, dass mit ihnen auch bei sehr kleinen Nadeln, insbesondere Mikronadeln, ein Faden sicher an der Nadel festgelegt werden kann.

Je nach zu verwendendem Nahtgut kann es vorteilhaft sein, wenn das Nadelöhr in Form einer im Querschnitt kreisförmigen, ovalen oder langlochartigen Durchbrechung des Nadelkörpers ausgebildet wird.

Vorzugsweise werden die Nadelspitze und/oder der gesamte Nadelkörper ganz oder teilweise mit einer Silikonschicht überzogen. Durch die Silikonschicht gleitet die Nadel noch besser durch Gewebe.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht eines langgestreckten zylindrischen Rohlings zur Ausbildung eines Nadelkörpers;
- Figur 2:: eine perspektivische Darstellung des Rohlings aus Figur 1 nach Einpressen von zwei Strukturprofilseitenflächenbereichen;
- Figur 3:: der mit einem Strukturprofil versehene Rohling aus Figur 2 nach Verbiegen des Grundkörpers;
- Figur 4:: eine perspektivische Ansicht des gebogenen Nadelkörpers aus Figur 3 nach Ausbilden zweier unprofilierter Seitenflächen;
- Figur 5:: eine schematische Darstellung des Nadelkörpers aus Figur 4 beim elektrochemischen Polieren der Nadelspitze;
- Figur 6:: eine schematische Darstellung der elektrochemisch polierten Nadelspitze; und
- Figur 7:: eine Schnittansicht längs Linie 7-7 durch den polierten Nadelkörper im Vergleich zu einer Schnittansicht durch den Nadelkörper vor dem elektrochemischen Polieren.

Nachfolgend wird eine bevorzugte Ausführungsform einer erfindungsgemäßen chirurgischen Nadel sowie eine Variante eines erfindungsgemäßen Verfahrens zum Herstellen einer chirurgischen Nadel beschrieben.

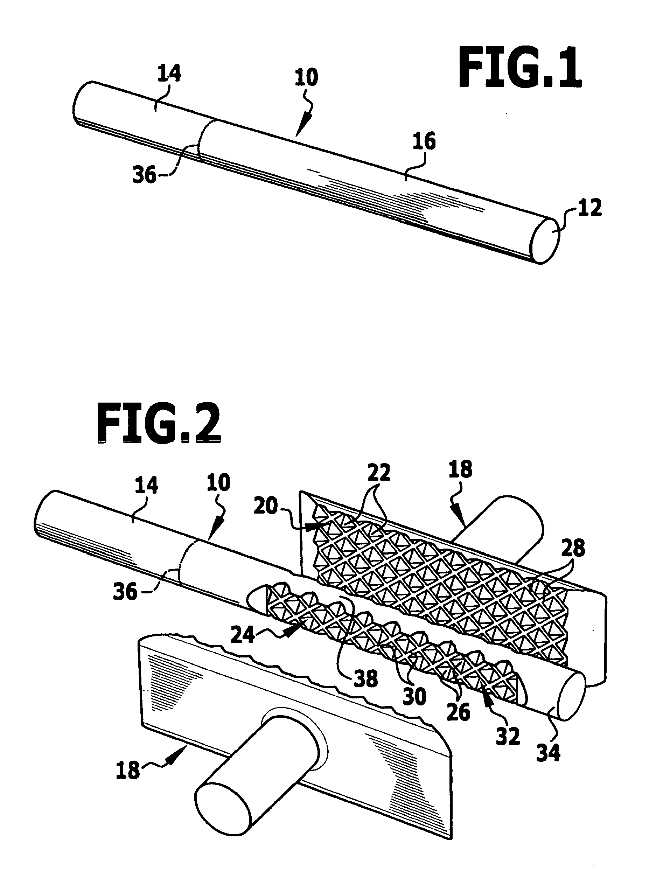

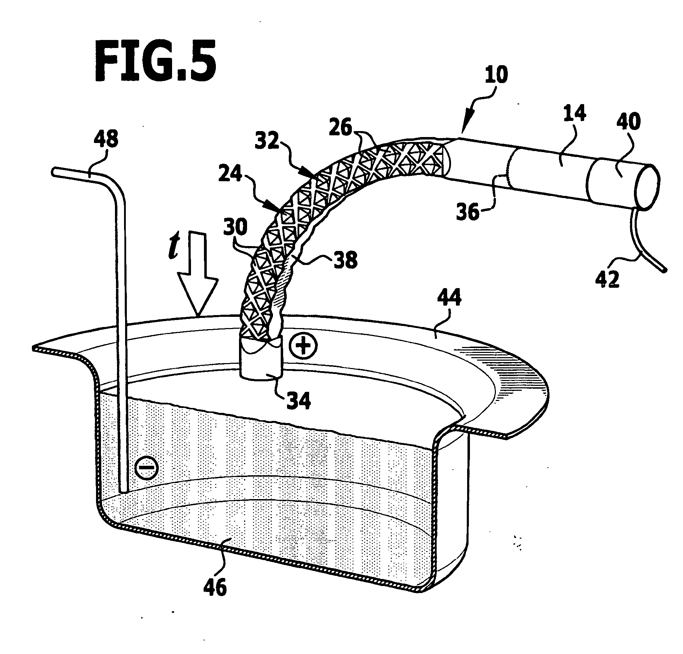

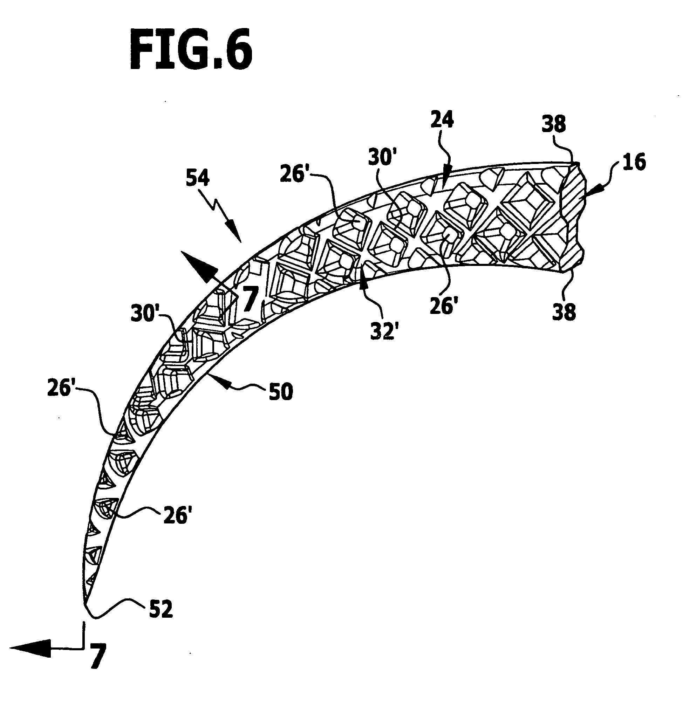

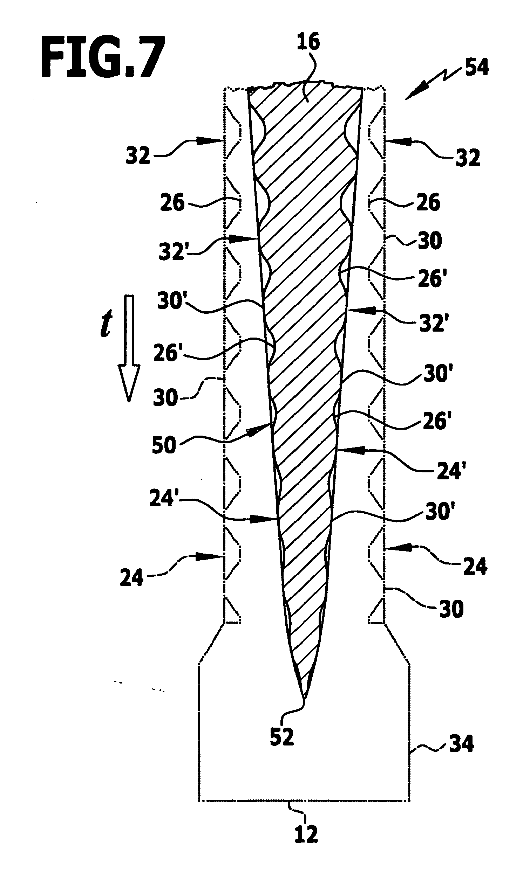

Bei dem im Zusammenhang mit den Figuren 1 bis 7 näher erläuterten Ausführungsbeispiel einer chirurgischen Nadel 54, die zumindest teilweise in den Figuren 6 und 7 dargestellt ist, handelt es sich insbesondere um eine mikrochirurgische Nadel 54 sowie ein Verfahren zum Herstellen einer chirurgischen Nadel 54. Ausgangspunkt des Verfahrens ist ein Rohling 10 aus einem langgestreckten Rundmaterial, beispielsweise einem Metalldraht, welcher einen kreisförmigen Querschnitt 12 aufweist. Etwa zwei Drittel der Länge des Rohlings 10 dienen als Hilfsschaft 14, der restliche Teil des Rohlings 14 bildet einen Grundkörper 16 für die chirurgische Nadel 54. Die Herstellung der chirurgischen Nadel ist jedoch auch ohne einen Hilfsschaft möglich. Der Rohling 10 zur Herstellung chirurgischer Mikro- und Makronadeln weist vorzugsweise einen Durchmesser in einem Bereich von 0,05 mm bis 1,5 mm auf, vorzugsweise in einem Bereich von 0,15 mm und 0,3 mm.

In einem ersten Schritt zur Herstellung der chirurgischen Nadel 54 werden mit zwei Bearbeitungswerkzeugen in Form von Stempeln 18, welche eine Profilfläche 20 mit einer Mehrzahl erhabener quadratischer Pyramiden 22 aufweisen, zwei Strukturprofilseitenflächenbereiche 24 am Rohling ausgebildet, und zwar derart, dass die Profilflächen 20 der beiden Stempel 18 aufeinander zu weisend gegen den Grundkörper 16 der Nadel 54 bildenden Abschnitt des Rohlings 10 gepresst werden, bis durch Materialverdichtung die Pyramiden 22 pyramidenartige Vertiefungen 26 im Grundkörper 16 ausgebildet haben. Die Pyramiden 22 sind durch eine Ebene definierende Trennstege 28 getrennt, ebenso wie die Vertiefungen 26 durch Trennstege 30. Die Trennstege 30 definieren eine äußere Oberfläche oder zumindest einen Teil derselben einer Strukturprofilseitenfläche 32 des Grundkörpers 16. Längsachsen der Trennstege 30 sind entweder parallel zur Längsachse des Rohlings 10 oder relativ zur Längsachse geneigt ausgerichtet. Bei dem in den Figuren dargestellten Ausführungsbeispiel sind Längsachsen der Trennstege bezogen auf die Längsachse des Rohlings um etwa 45° geneigt.

Durch die Verwendung zweier Stempel 18 werden am Grundkörper 16 zwei Strukturprofilseitenflächen 32 ausgebildet, die in entgegengesetzten Richtungen voneinander weg weisen.

Die die Profilfläche 20 ausbildenden Vorsprünge, die beispielsweise in Form der beschriebenen erhabenen quadratischen Pyramiden 22 ausgebildet sein können, sind vorzugsweise regelmäßig angeordnet, beispielsweise in einem regelmäßigen Muster mit einer mehrzähligen Symmetrie. Bei dem in den Figuren dargestellten Ausführungsbeispiel weist die Profilfläche 20 eine vierzählige Symmetrie auf.

Denkbar wären aber auch drei- oder sechszählige Symmetrien mit entsprechend geformten Vorsprüngen. Durch den beschriebenen Pressvorgang erhalten auch die Strukturprofilseitenflächenbereiche 24 ein entsprechendes symmetrisches Erscheinungsbild.

Zur Ausbildung chirurgischer Nadeln, sowohl von Mikronadeln als auch von Makronadeln, werden vorzugsweise Stempel 18 verwendet, deren Pyramiden 20 Seitenkanten mit Abmessungen in einem Bereich von 0,02 mm bis 1 mm haben, insbesondere auch in einem Bereich von 0,07 mm bis 0,1 mm, vorzugsweise 0,085 mm. Die Trennstege 28 und 30 zwischen den Pyramiden 22 und den Vertiefungen 26 weisen eine Breite in einem Bereich von 0,009 mm bis 1 mm, insbesondere auch in einem Bereich von 0,025 mm bis 0,04 mm auf, vorzugsweise eine Breite von 0,03 mm.

Der durch die Stempel 18 gebildete Strukturprofilseitenflächenbereich 24 erstreckt sich nicht über die gesamte Länge des Grundkörpers 16, sondern endet vielmehr etwas beabstandet von einem distalen Ende 34 des Grundkörpers 16 sowie proximalseitig von einem Grenzbereich 36 zwischen dem Grundkörper 16 und dem Hilfsschaft 14, die einstückig miteinander verbunden sind und nach Abschluss aller Bearbeitungsschritte voneinander durch Trennung im Grenzbereich getrennt werden können.

Eine Höhe der Pyramiden 22 bezogen auf die von den Trennstegen 28 definierte Ebene liegt in einem Bereich von 0,04 mm bis 0,1 mm. Dementsprechend ergibt sich für die eingepressten Vertiefungen 26 eine Tiefe bezogen auf eine von den Trennstegen 30 definierte äußere Oberfläche von ebenfalls in einem Bereich von 0,04 mm bis 0,1 mm.

Durch das Ausbilden der Strukturprofilseitenflächenbereiche 24 weist der Grundkörper 16 zwei Strukturprofilseitenflächen 32 auf sowie zwei diese miteinander verbindende unprofilierte Seitenflächen 38, die sich in Längsrichtung des Grundkörpers 16 erstrecken und vom Grundkörper 16 weg weisend konvex gekrümmt sind. Durch die jeweils eine Profilfläche 20 aufweisenden Stempel 18 sind die Strukturprofilseitenflächenbereiche 24 eben beziehungsweise im Wesentlichen eben.

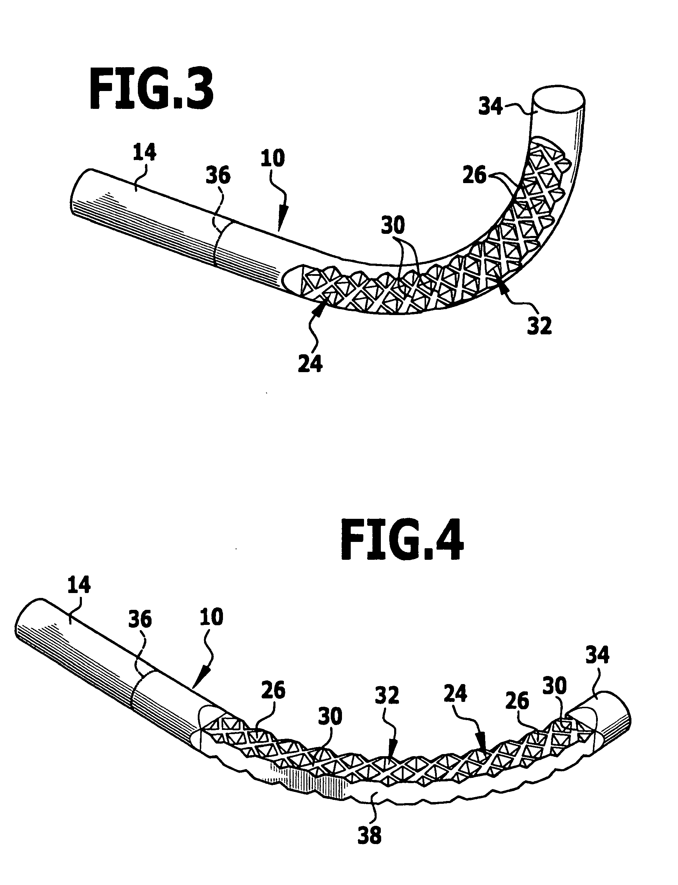

Ein optionaler Verfahrensschritt zum Herstellen der chirurgischen Nadel besteht darin, den Grundkörper 16 zu biegen. Hierzu gibt es mehrere Möglichkeiten. Zum einen kann der Grundkörper so gebogen werden, dass ein unprofilierter, sich an den Grenzbereich 36 anschließender Bereich des Grundkörpers 16 unverbogen bleibt, der Grundkörper 16 nur im Bereich der ausgebildeten Strukturprofilseitenflächenbereiche 24 gekrümmt ist. Ein Krümmungsradius des Grundkörpers kann in einem Bereich von 0,5 mm bis 50 mm liegen. Das distale Ende 34 des Grundkörpers 16 bleibt ebenfalls unverbogen und bildet somit einen geradlinigen zylindrischen Abschnitt. Das Biegen des Grundkörpers 36 kann derart vorgesehen werden, dass nach dem Biegen des Grundkörpers 16 die Trennstege 30 der Strukturprofilseitenflächenbereiche 24 nach wie vor zwei zueinander parallele Ebenen definieren. Eine derartige Abbiegungsform ist beispielsweise in den Figuren 3 und 4 dargestellt. Es wäre jedoch auch denkbar, dass die unprofilierten Seitenflächen 38, die aufgrund ihrer Krümmung zwei zueinander parallele, nicht dargestellte Tangentialebenen berühren, nach dem Biegen des Grundkörpers 16 nach wie vor die selben beiden Tangentialebenen definieren beziehungsweise diese berühren. Diese Möglichkeit den Grundkörper 16 zu biegen, ist in den Figuren nicht dargestellt.

Nach dem optionalen Schritt des Biegens des Grundkörpers 16 können die Seitenflächen 38 optional und ebenfalls durch Pressen mit zwei nicht dargestellten Stempeln profiliert werden. Beispielsweise können Stempel mit ebenen Stempelflächen verwendet werden, so dass die zunächst konkav vom Grundkörper 16 weg weisend gekrümmten Seitenflächen 38 nach dem Pressvorgang jeweils ebene Flächen definieren, was beispielsweise bei ungekrümmten Grundkörpern 16 einfach möglich ist sowie bei gekrümmten Grundkörpern, bei denen die Seitenflächen 38 nach dem Biegen immer noch die beiden gleichen zu einander parallelen Ebenen berühren. In diesen Fällen können Stempel mit ebenen Stempelflächen verwendet werden.

Wird der Grundkörper 16 jedoch, wie oben beschrieben und in den Figuren 3 und 4 dargestellt, derart gebogen, dass die von den Trennstegen 30 gebildeten Ebenen nach dem Biegen unverändert bleiben, dann werden zum Profilieren beziehungsweise Abflachen der Seitenflächen 38 des Grundkörpers 16 zwei Stempel mit gekrümmten Stempelflächen verwendet, die einerseits einen inneren Radius des gebogenen Grundkörpers 16 und andererseits einen äußeren Radius des gebogenen Grundkörpers 16 definieren.

Der mit Strukturprofilseitenflächen 24 versehene sowie optional gekrümmte und optional mit profilierten Seitenflächen 38 versehene Rohling 10 weist, wie in den Figuren 2 bis 4 dargestellt, noch keine Nadelspitze auf. Diese könnte zwar durch mechanische Bearbeitung, beispielsweise Abschleifen, ausgebildet werden. Dies führt jedoch dazu, dass die Vertiefungen 26 abgeschliffen würden, so dass im Bereich der Nadelspitze kein Profil mehr vorhanden wäre. Daher wird eine Nadelspitze 50 in einem weiteren Bearbeitungsschritt durch elektrochemisches Polieren geformt. Hierzu wird der Hilfsschaft 14 mit einer positiven Elektrode verbunden, die über eine Verbindungsleitung 42 mit einem nicht dargestellten Gleichspannungsnetzgerät verbunden wird. Ein mit einem geeigneten Elektrolyt 46 gefüllter topfartiger Behälter 44 bildet ein Elektrolysebad. Zum elektrochemischen Polieren wird eine negative Elektrode 48 in den Elektrolyt 46 eingetaucht, welche beispielsweise mit einem negativen Pol des nicht dargestellten Netzgerätes verbunden ist. Zur Ausbildung der Nadelspitze 50 wird nunmehr das distale Ende 34 des Grundkörpers 16 in den Elektrolyt 46 eingetaucht und ein Stromfluss eingestellt. Das Eintauchen kann sukzessive geschehen, das heißt der Grundkörper 16 wird langsam über einen bestimmten Zeitraum hinweg in den Elektrolyt 46 eingetaucht, und zwar so weit, wie die eigentliche Nadelspitze 50 lang werden soll. Es wäre aber auch denkbar, das distale Ende 34 des Grundkörpers 16 sofort bis zur gewünschten fertigen Länge der Nadelspitze 50 in den Elektrolyt 46 einzutauchen.

Eine besonders gute Spitzenform lässt sich erreichen, wenn sichergestellt ist, dass das distale Ende 34 vor dem elektrochemischen Polieren einen runden Querschnitt aufweist. Je runder der Querschnitt am distalen Ende 34 ist, um so besser die Qualität, das Design und die Stabilität der Nadelspitze 50.

Durch das elektrochemische Polieren wird Material vom Grundkörper 16 abgetragen, und zwar in dem Bereich, in dem der Grundkörper 16 in den Elektrolyt 46 eintaucht. Anders als beim mechanischen Polieren wird beim elektrochemischen Polieren auch Material aus den Vertiefungen abgetragen. Dies hat zur Folge, dass im Bereich der dann ausgebildeten Nadelspitze 50 die Vertiefungen 26 ebenfalls teilweise abgetragen werden. Daher bleibt auch die Nadelspitze 50 bis zu ihrem distalen Ende 52 profiliert, auch wenn eine Tiefe der Vertiefungen 26 bezogen auf eine von den die in ihrer Form veränderten Vertiefungen 26' trennenden Trennstege 30' in Richtung auf das Ende 52 hin etwas abnimmt

Durch das elektrochemische Polieren vermischen sich zudem die ursprünglich eingeprägten Formen der Vertiefungen 26 etwas, so dass am Ende ein verwaschenes oder auch als ausgerundetes Profil der Nadelspitze 50 beschreibbares Strukturprofil entsteht.

Aufgrund des Materialabtrags im Bereich der Nadelspitze 50 verändern sich auch die Trennstege 30, was beispielhaft durch Figur 7 angedeutet ist. Die nach dem elektrochemischen Polieren verbleibenden Vertiefungen 26' sowie die verbleibenden Trennstege 30' stimmen nicht mehr mit den ursprünglichen Vertiefungen 26 und den ursprünglichen Trennstegen 30 überein, wie sich im Vergleich der im Schnitt dargestellten fertigen Nadelspitze 50 zum mit Strukturprofilseitenflächenbereichen 24 versehenen Grundkörper 16 ergibt.

Die Vertiefungen 26 können auch eine Form aufweisen, wie sie Profilierungen bei Golfbällen entsprechen, das heißt die Seitenflächen der Vertiefungen 26 sind etwas vom Grundkörper 16 weg gewölbt. Dies lässt sich beispielsweise erreichen durch Pyramiden der Stempel 18, die konkave Seitenflächen aufweisen. In der Draufsicht derartiger eingeprägter Vertiefungen haben diese ein kreuzsternfömiges Design, das der strukturierten Oberfläche eines Golfballs nachempfunden ist.

In einem vierten, jedoch optionalen Bearbeitungsschritt, kann die Nadelspitze 50 oder aber auch der gesamte Körper der Nadel 54 silikonisiert werden, das heißt mit einer Schicht aus Silikon bedeckt. Durch die Ausbildung von voneinander getrennten Vertiefungen 26 können diese als Silikonreservoir dienen, so dass sich mit erfindungsgemäß hergestellten Nadeln mehrere Durchstiche nacheinander an Gewebe vornehmen lassen, ohne dass ein aufgrund des Silikonisierens verbessertes Gleitverhalten des Nadelkörpers sich verschlechtert.

Aufgrund des vorgesehenen Strukturprofils auf mindestens einer Strukturprofilseitenfläche des Nadelkörpers verbessert sich das Gleitverhalten erfindungsgemäßer Nadeln gegenüber herkömmlichen Nadeln signifikant. Ferner verbessert sich die Biegesteifigkeit gegenüber herkömmlichen Nadeln deutlich. Diese deutlichen Verbesserungen gegenüber aus dem Stand der Technik bekannten Nadeln haben den Vorteil, dass erfindungsgemäß ausgebildete Nadeln mit weniger Kraftaufwand zum Durchstechen von Gewebe eingesetzt werden können und zudem die Gefahr eines Brechens der Nadeln beim chirurgischen Einsatz vermindert ist. Die Verbesserung der Biegesteifigkeit der Nadel wird durch Kaltverfestigung des Materials, aus dem der Rohling 10 hergestellt ist, durch das Einpressen der Strukturprofilseitenflächenbereiche 24 erreicht.

Wie bereits oben dargelegt, ist es nicht zwingend erforderlich, die erfindungsgemäße Nadel aus einem langgestreckten zylindrischen Rohling zu formen, beispielsweise aus einem geraden, runden Drahtstift. Es ist vielmehr auch denkbar, die Nadel insgesamt durch Spritzgießen, beispielsweise durch Metallspritzgießen (MIM - Metal Injection Molding), Pulverspritzgießen (PIM - Powder Injection Molding) oder auch durch Kunststoffspritzgießen herzustellen. Es wäre auch möglich, die Nadel aus einer Keramik herzustellen, die durch Einfüllen des Ausgangsmaterials in eine Form und anschließendes Sintern hergestellt wird. Optional wäre es auch möglich, einen Rohling, wie oben beschrieben, durch die verschiedenen Spritzgusstechniken oder durch Sintern herzustellen und erst anschließend die Spitze, wie oben im Zusammenhang mit dem in den Figuren dargestellten Ausführungsbeispiel, durch elektrochemisches Polieren die Nadelspitze auszubilden.

## Patentansprüche

1. Chirurgische Nadel (54) mit einem langgestreckten Nadelkörper (16) und einer an einem distalen Ende (34) des Nadelkörpers (16) ausgebildeten Nadelspitze (50, 52), wobei der Nadelkörper (16) insbesondere aus einem langgestreckten zylindrischen Rohling (10) hergestellt ist, wobei der Nadelkörper (16) mindestens eine Strukturprofilseitenfläche (32) aufweist, die mindestens einen mit einem Strukturprofil versehenen Strukturprofilseitenflächenbereich (24) umfasst, und wobei sich der mindestens eine Strukturprofilseitenflächenbereich (24) bis zur Nadelspitze (50, 52) erstreckt, **dadurch gekennzeichnet, dass** das Strukturprofil in Form eines eingeprägten Profils ausgebildet ist und eine Mehrzahl von eingeprägten Vertiefungen (26) aufweist.

2. Nadel nach Anspruch 1, **dadurch gekennzeichnet, dass** der mindestens eine Strukturprofilseitenflächenbereich (24) mindestens die Hälfte der mindestens einen Strukturprofilseitenfläche (32) bildet.

3. Nadel nach einem der voranstehenden Ansprüche, **dadurch gekennzeichnet, dass** der Nadelkörper (16) zwei Strukturprofilseitenflächen (24) aufweist und dass die zwei Strukturprofilseitenflächen (24) in entgegengesetzten Richtungen voneinander weg weisen.

4. Nadel nach einem der voranstehenden Ansprüche, **dadurch gekennzeichnet, dass** der Nadelkörper (16) im Querschnitt eine rechteckige oder quadratische oder im Wesentlichen rechteckige oder quadratische Form aufweist.

5. Nadel nach einem der voranstehenden Ansprüche, **dadurch gekennzeichnet, dass** der Nadelkörper (16) vier ebene oder im Wesentlichen ebene Seitenflächen (32, 38) aufweist und dass eine der Seitenflächen (32) eine Strukturprofilseitenfläche (32) ist.

6. Nadel nach einem der voranstehenden Ansprüche, **dadurch gekennzeichnet, dass** der Nadelkörper (16) mindestens eine an die mindestens eine Strukturprofilseitenfläche (32) angrenzende unprofilierte Seitenfläche (38) aufweist.

7. Nadel nach Anspruch 6, **dadurch gekennzeichnet, dass** die mindestens eine unprofilierte Seitenfläche (38) vom Nadelkörper (16) weg weisend konvex gekrümmt ist.

8. Nadel nach Anspruch 6 oder 7, **dadurch gekennzeichnet, dass** zwei unprofilierte oder im Wesentlichen unprofilierte Seitenflächen (38) vorgesehen sind.

9. Nadel nach Anspruch 8, **dadurch gekennzeichnet, dass** die zwei unprofilierten oder im Wesentlichen unprofilierten Seitenflächen (38) in entgegengesetzten Richtungen voneinander weg weisen.

10. Nadel nach einem der Ansprüche 6 bis 8, **dadurch gekennzeichnet, dass** der Nadelkörper (16) zwei Strukturprofilseitenflächen (32) aufweist und dass zwei die Strukturprofilseitenflächen (32) miteinander verbindende unprofilierte oder im Wesentlichen unprofilierte Seitenflächen (38) vorgesehen sind.

11. Nadel nach einem der voranstehenden Ansprüche, **dadurch gekennzeichnet, dass** der Nadelkörper (16) gebogen ist.

12. Nadel nach einem der voranstehenden Ansprüche, **dadurch gekennzeichnet, dass** die mindestens eine Strukturprofilseitenfläche (32) eine äußere Oberfläche definiert und dass sich die Mehrzahl von Vertiefungen (26) von der äußeren Oberfläche in den Nadelkörper (16) hinein erstreckt.

13. Nadel nach Anspruch 12, **dadurch gekennzeichnet, dass** eine Tiefe der Vertiefungen (26) bezogen auf die äußere Oberfläche der mindestens einen Strukturprofilseitenfläche (32) ausgehend von einem proximalen Ende des mindestens einen Strukturprofilseitenflächenbereichs (24) in Richtung auf die Nadelspitze (52) hin abnimmt.

14. Nadel nach einem der voranstehenden Ansprüche, **dadurch gekennzeichnet, dass** die Mehrzahl von Vertiefungen (26) ganz oder teilweise pyramidenförmig ausgebildet ist.

15. Nadel nach Anspruch 14, **dadurch gekennzeichnet, dass** die Mehrzahl von Vertiefungen (26) ganz oder teilweise in Form quadratischer Pyramiden (26) ausgebildet ist.

16. Nadel nach einem der voranstehenden Ansprüche, **dadurch gekennzeichnet, dass** die Mehrzahl von Vertiefungen (26) eingepresst ist.

17. Nadel nach einem der Ansprüche 1 bis 16, **dadurch gekennzeichnet, dass** der Nadelkörper (16) durch Spritzgießen hergestellt ist.

18. Nadel nach Anspruch 17, **dadurch gekennzeichnet, dass** der Nadelkörper (16) durch Metallspritzgießen (MIM - Metal Injection Molding), Pulverspritzgießen (PIM - Powder Injection Molding) oder durch Kunststoffspritzgießen hergestellt ist.

19. Nadel nach einem der voranstehenden Ansprüche, **dadurch gekennzeichnet, dass** die Nadel (54) aus einem Metall, einer Keramik oder aus Kunststoff hergestellt ist.

20. Nadel nach einem der voranstehenden Ansprüche, **dadurch gekennzeichnet, dass** die Nadelspitze (50) durch elektrochemisches Polieren hergestellt ist.

21. Nadel nach einem der voranstehenden Ansprüche, **dadurch gekennzeichnet, dass** die Nadel (54) an einem proximalen Ende mindestens ein Nadelöhr, ein Sackloch oder eine Kerbe aufweist.

22. Nadel nach Anspruch 21, **dadurch gekennzeichnet, dass** das Nadelöhr in Form einer im Querschnitt kreisförmigen, ovalen oder langlochartigen Durchbrechung des Nadelkörpers (16) ausgebildet ist.

23. Nadel nach einem der voranstehenden Ansprüche, **dadurch gekennzeichnet, dass** die Nadelspitze (50) und/oder der gesamte Nadelkörper ganz oder teilweise mit einer Silikonschicht überzogen sind.

24. Verfahren zum Herstellen einer chirurgischen Nadel (54) mit einem langgestreckten Nadelkörper und einer an einem distalen Ende (34) des Nadelkörpers (16) ausgebildeten Nadelspitze (50, 52), wobei der Nadelkörper (16) insbesondere aus einem langgestreckten zylindrischen Rohling (10) hergestellt werden kann, wobei der Nadelkörper (16) mit mindestens einer Strukturprofilseitenfläche (32) versehen wird, wobei die mindestens eine Strukturproflseitenfläche (32) zur Ausbildung mindestens eines Strukturprohlseitenflächenbereichs (24) mit einem Strukturprofil derart versehen wird, dass sich der mindestens eine Strukturprofilseitenflächenbereich (24) bis zur Nadelspitze (50, 52) erstreckt, **dadurch gekennzeichnet, dass** das Strukturprofil in Form eines eingeprägten Profils ausgebildet wird, welches eine Mehrzahl von eingeprägten Vertiefungen (26) aufweist,.

25. Verfahren nach Anspruch 24, **dadurch gekennzeichnet, dass** der mindestens eine Strukturprofilseitenflächenbereich (24) derart ausgebildet wird, dass er mindestens die Hälfte der mindestens einen Strukturprofilseitenfläche (32) bildet.

26. Verfahren nach einem der Ansprüche 24 oder 25, **dadurch gekennzeichnet, dass** der Nadelkörper (16) mit zwei Strukturprofilseitenflächen (32) versehen wird, so dass die zwei Strukturprofilseitenflächen (32) in entgegengesetzten Richtungen voneinander weg weisen.

27. Verfahren nach einem der Ansprüche 24 bis 26, **dadurch gekennzeichnet, dass** der Nadelkörper (16) derart ausgebildet wird, dass er im Querschnitt eine rechteckige oder quadratische oder im Wesentlichen rechteckige oder quadratische Form aufweist.

28. Verfahren nach einem der Ansprüche 24 bis 27, **dadurch gekennzeichnet, dass** der Nadelkörper (16) derart ausgebildet wird, dass er vier ebene oder im Wesentlichen ebene Seitenflächen (32, 38) aufweist und dass mindestens eine der Seitenflächen (32) eine Strukturprofilseitenfläche (32) ist.

29. Verfahren nach einem der Ansprüche 24 bis 28, **dadurch gekennzeichnet, dass** der Nadelkörper (16) derart ausgebildet wird, dass er mindestens eine an die mindestens eine Strukturprohlseitenfläche (32) angrenzende unprofilierte Seitenfläche (38) aufweist.

30. Verfahren nach Anspruch 29, **dadurch gekennzeichnet, dass** die mindestens eine unprofilierte Seitenfläche (38) vom Nadelkörper (16) weg weisend konvex gekrümmt ausgebildet wird.

31. Verfahren nach Anspruch 29 oder 30, **dadurch gekennzeichnet, dass** zwei unprofilierte oder im Wesentlichen unprofilierte Seitenflächen (38) ausgebildet werden.

32. Verfahren nach Anspruch 31, **dadurch gekennzeichnet, dass** die zwei unprofilierten oder im Wesentlichen unprofilierten Seitenflächen (38) in entgegengesetzten Richtungen voneinander weg weisend ausgebildet werden.

33. Verfahren nach einem der Ansprüche 29 bis 32, **dadurch gekennzeichnet, dass** der Nadelkörper (16) derart ausgebildet wird, dass er zwei Strukturprofilseitenflächen (32) aufweist und dass zwei die Strukturprofilseitenflächen (32) miteinander verbindende unprofilierte oder im Wesentlichen unprofilierte Seitenflächen (38) ausgebildet werden.

34. Verfahren nach einem der Ansprüche 24 bis 33, **dadurch gekennzeichnet, dass** der Nadelkörper (16) nach der Ausbildung des mindestens einen Strukturprofilseitenflächenbereichs (24) gebogen wird.

35. Verfahren nach einem der Ansprüche 24 bis 34, **dadurch gekennzeichnet, dass** die mindestens eine Strukturprofilseitenfläche (32) eine äußere Oberfläche definiert und dass die Mehrzahl von Vertiefungen (26) von der äußeren Oberfläche in den Nadelkörper (16) hinein geformt werden.

36. Verfahren nach Anspruch 35, **dadurch gekennzeichnet, dass** eine Tiefe der Vertiefungen (26) bezogen auf die äußere Oberfläche der mindestens einen Strukturprofilseitenfläche (32) ausgehend von einem proximalen Ende des mindestens einen Strukturprofilseitenflächenbereichs (24) in Richtung auf die Nadelspitze (50, 52) hin abnehmend ausgebildet wird.

37. Verfahren nach einem der Ansprüche 24 bis 36, **dadurch gekennzeichnet, dass** die Mehrzahl von Vertiefungen (26) ganz oder teilweise pyramidenförmig ausgebildet wird.

38. Verfahren nach Anspruch 37, **dadurch gekennzeichnet, dass** die Mehrzahl von Vertiefungen (26) ganz oder teilweise in Form quadratischer Pyramiden (26) ausgebildet wird.

39. Verfahren nach einem der Ansprüche 24 bis 38, **dadurch gekennzeichnet, dass** die Mehrzahl von Vertiefungen (26) eingepresst werden.

40. Verfahren nach einem der Ansprüche 24 bis 38, **dadurch gekennzeichnet, dass** der Nadelkörper (16) durch Spritzgießen hergestellt wird.

41. Verfahren nach Anspruch 40, **dadurch gekennzeichnet, dass** der Nadelkörper (16) durch Metallspritzgießen (MIM - Metal Injection Molding), Pulverspritzgießen (PIM - Powder Injection Molding) oder durch Kunststoffspritzgießen hergestellt wird.

42. Verfahren nach einem der Ansprüche 24 bis 41, **dadurch gekennzeichnet, dass** die Nadel (54) aus einem Metall, aus Kunststoff oder in Form einer Keramik durch Sintern hergestellt wird.

43. Verfahren nach einem der Ansprüche 24 bis 42, **dadurch gekennzeichnet, dass** die Nadelspitze (50) durch elektrochemisches Polieren hergestellt wird.

44. Verfahren nach einem der Ansprüche 24 bis 43, **dadurch gekennzeichnet, dass** die Nadel (54) an einem proximalen Ende mit mindestens einem Nadelöhr, einem Sackloch oder einer Kerbe versehen wird.

45. Verfahren nach Anspruch 44, **dadurch gekennzeichnet, dass** das Nadelöhr in Form einer im Querschnitt kreisförmigen, ovalen oder langlochartigen Durchbrechung des Nadelkörpers (16) ausgebildet wird.

46. Verfahren nach einem der Ansprüche 24 bis 45, **dadurch gekennzeichnet, dass** die Nadelspitze (50, 52) und/oder der gesamte Nadelkörper (16) ganz oder teilweise mit einer Silikonschicht überzogen werden.

## Claims

1. Surgical needle (54) comprising an elongate needle body (16) and a needle point (50, 52) formed on a distal end (34) of the needle body (16), wherein the needle body (16) is manufactured in particular from an elongate cylindrical blank (10), wherein the needle body (16) has at least one textured-profile lateral face (32), which comprises at least one textured-profile lateral-face region (24) provided with a textured profile, and wherein the at least one textured-profile lateral-face region (24) extends as far as the needle point (50, 52), **characterized in that** the textured profile is designed in the form of an embossed profile and comprises a plurality of embossed indentations (26).

2. Needle according to claim 1, **characterized in that** the at least one textured-profile lateral-face region (24) forms at least half of the at least one textured-profile lateral face (32).

3. Needle according to one of the preceding claims, **characterized in that** the needle body (16) has two textured-profile lateral faces (24) and that the two textured-profile lateral faces (24) face in opposite directions away from one another.

4. Needle according to one of the preceding claims, **characterized in that** the needle body (16) in cross section has a rectangular or square or substantially rectangular or square shape.

5. Needle according to one of the preceding claims, **characterized in that** the needle body (16) has four flat or substantially flat lateral faces (32, 38) and that one of the lateral faces (32) is a textured-profile lateral face (32).

6. Needle according to one of the preceding claims, **characterized in that** the needle body (16) has at least one non-profiled lateral face (38) adjoining the at least one textured-profile lateral face (32).

7. Needle according to claim 6, **characterized in that** the at least one non-profiled lateral face (38) has a convex curvature in a direction away from the needle body (16).

8. Needle according to claim 6 or 7, **characterized in that** two non-profiled or substantially non-profiled lateral faces (38) are provided.

9. Needle according to claim 8, **characterized in that** the two non-profiled or substantially non-profiled lateral faces (38) face in opposite directions away from one another.

10. Needle according to one of claims 6 to 8, **characterized in that** the needle body (16) has two textured-profile lateral faces (32) and that two non-profiled or substantially non-profiled lateral faces (38) are provided, which connect the textured-profile lateral faces (32) to one another.

11. Needle according to one of the preceding claims, **characterized in that** the needle body (16) is bent.

12. Needle according to one of the preceding claims, **characterized in that** the at least one textured-profile lateral face (32) defines an outer surface and that the plurality of indentations (26) extend from the outer surface into the needle body (16).

13. Needle according to claim 12, **characterized in that** a depth of the indentations (26) relative to the outer surface of the at least one textured-profile lateral face (32) decreases from a proximal end of the at least one textured-profile lateral-face region (24) in the direction of the needle point (52).

14. Needle according to one of the preceding claims, **characterized in that** the plurality of indentations (26) are entirely or partially of a pyramidal design.

15. Needle according to claim 14, **characterized in that** the plurality of indentations (26) are entirely or partially designed in the form of square pyramids (26).

16. Needle according to one of the preceding claims, **characterized in that** the plurality of indentations (26) are impressed.

17. Needle according to one of claims 1 to 16, **characterized in that** the needle body (16) is manufactured by injection moulding.

18. Needle according to claim 17, **characterized in that** the needle body (16) is manufactured by metal injection moulding (MIM), powder injection moulding (PIM) or by plastic injection moulding.

19. Needle according to one of the preceding claims, **characterized in that** the needle (54) is manufactured from a metal, a ceramic material or from plastics material.

20. Needle according to one of the preceding claims, **characterized in that** the needle point (50) is manufactured by electrochemical polishing.

21. Needle according to one of the preceding claims, **characterized in that** the needle (54) at a proximal end has at least one eye, blind hole or notch.

22. Needle according to claim 21, **characterized in that** the eye is designed in the form of a through opening in the needle body (16) that is circular, oval or elongate-hole-like in cross section.

23. Needle according to one of the preceding claims, **characterized in that** the needle point (50) and/or the entire needle body is entirely or partially coated with a silicone layer.

24. Method of manufacturing a surgical needle (54) comprising an elongate needle body and a needle point (50, 52) formed at a distal end (34) of the needle body (16), wherein the needle body (16) may be manufactured in particular from an elongate cylindrical blank (10), wherein the needle body (16) is provided with at least one textured-profile lateral face (32), wherein the at least one textured-profile lateral face (32) for forming at least one textured-profile lateral-face region (24) is provided with a textured profile in such a way that the at least one textured-profile lateral-face region (24) extends as far as the needle point (50, 52), **characterized in that** the textured profile is designed in the form of an embossed profile and comprises a plurality of embossed indentations (26).

25. Method according to claim 24, **characterized in that** the at least one textured-profile lateral-face region (24) is designed in such a way that it forms at least half of the at least one textured-profile lateral face (32).

26. Method according to one of claims 24 or 25, **characterized in that** the needle body (16) is provided with two textured-profile lateral faces (32) such that the two textured-profile lateral faces (32) face in opposite directions away from one another.

27. Method according to one of claims 24 to 26, **characterized in that** the needle body (16) is designed in such a way that in cross section it has a rectangular or square or substantially rectangular or square shape.

28. Method according to one of claims 24 to 27, **characterized in that** the needle body (16) is designed in such a way that it has four flat or substantially flat lateral faces (32, 38) and that at least one of the lateral faces (32) is a textured-profile lateral face (32).

29. Method according to one of claims 24 to 28, **characterized in that** the needle body (16) is designed in such a way that it has at least one non-profiled lateral face (38) adjoining the at least one textured-profile lateral face (32).

30. Method according to claim 29, **characterized in that** the at least one non-profiled lateral face (38) is designed with a convex curvature in a direction away from the needle body (16).

31. Method according to claim 29 or 30, **characterized in that** two non-profiled or substantially non-profiled lateral faces (38) are formed.

32. Method according to claim 31, **characterized in that** the two non-profiled or substantially non-profiled lateral faces (38) are designed facing in opposite directions away from one another.

33. Method according to one of claims 29 to 32, **characterized in that** the needle body (16) is designed in such a way that it has two textured-profile lateral faces (32) and that two non-profiled or substantially non-profiled lateral faces (38) are formed, which connect the textured-profile lateral faces (32) to one another.

34. Method according to one of claims 24 to 33, **characterized in that** the needle body (16) after the formation of the at least one textured-profile lateral-face region (24) is bent.

35. Method according to one of claims 24 to 34, **characterized in that** the at least one textured-profile lateral face (32) defines an outer surface and that the plurality of indentations (26) are formed from the outer surface into the needle body (16).

36. Method according to claim 35, **characterized in that** a depth of the indentations (26) relative to the outer surface of the at least one textured-profile lateral face (32) is designed to decrease from a proximal end of the at least one textured-profile lateral-face region (24) in the direction of the needle point (50, 52).

37. Method according to one of claims 24 to 36, **characterized in that** the plurality of indentations (26) are entirely or partially of a pyramidal design.

38. Method according to claim 37, **characterized in that** the plurality of indentations (26) are designed entirely or partially in the form of square pyramids (26).

39. Method according to one of claims 24 to 38, **characterized in that** the plurality of indentations (26) are impressed.

40. Method according to one of claims 24 to 38, **characterized in that** the needle body (16) is manufactured by injection moulding.

41. Method according to claim 40, **characterized in that** the needle body (16) is manufactured by metal injection moulding (MIM), power injection moulding (PIM) or by plastic injection moulding.

42. Method according to one of claims 24 to 41, **characterized in that** the needle (54) is manufactured from a metal, from plastics material or in the form of a ceramic product by sintering.

43. Method according to one of claims 24 to 42, **characterized in that** the needle point (50) is manufactured by electrochemical polishing.

44. Method according to one of claims 24 to 43, **characterized in that** the needle (54) at a proximal end is provided with at least one eye, blind hole or notch.

45. Method according to claim 44, **characterized in that** the eye is designed in the form of a through opening in the needle body (16) that is circular, oval or elongate-hole-like in cross section.

46. Method according to one of claims 24 to 45, **characterized in that** the needle point (50, 52) and/or the entire needle body (16) is entirely or partially coated with a silicone layer.

## Revendications

1. Aiguille chirurgicale (54) présentant un corps d'aiguille (16) allongé et une pointe d'aiguille (50, 52) formée à une extrémité distale (34) du corps d'aiguille (16), le corps d'aiguille (16) étant notamment fabriqué à partir d'une ébauche brute cylindrique (10) allongée, le corps d'aiguille (16) présentant au moins une surface latérale à profil structuré (32), qui comprend au moins une zone de surface latérale à profil structuré (24) pourvue d'un profil structuré, et ladite au moins une zone de surface latérale à profil structuré (24) s'étendant jusqu'à la pointe d'aiguille (50, 52),
**caractérisée en ce que** le profil structuré est réalisé sous la forme d'un profil obtenu par estampage et présente un grand nombre d'empreintes en creux (26) obtenues par estampage.

2. Aiguille selon la revendication 1, **caractérisée en ce que** ladite au moins une zone de surface latérale à profil structuré (24) forme au moins la moitié de ladite au moins une surface latérale à profil structuré (32).

3. Aiguille selon l'une des revendications précédentes, **caractérisée en ce que** le corps d'aiguille (16) présente deux surfaces latérales à profil structuré (24), et **en ce que** les deux surfaces latérales à profil structuré (24) ont des orientations respectives pointant dans des directions opposées.

4. Aiguille selon l'une des revendications précédentes, **caractérisée en ce que** le corps d'aiguille (16) présente, en section transversale, une forme rectangulaire ou carrée, ou sensiblement rectangulaire ou carrée.

5. Aiguille selon l'une des revendications précédentes, **caractérisée en ce que** le corps d'aiguille (16) présente quatre surfaces latérales planes ou sensiblement planes (32, 38), et **en ce que** l'une des surfaces latérales (32) est une surface latérale à profil structuré (32).

6. Aiguille selon l'une des revendications précédentes, **caractérisée en ce que** le corps d'aiguille (16) présente au moins une surface latérale non profilée (38) adjacente à ladite au moins une surface latérale à profil structuré (32).

7. Aiguille selon la revendication 6, **caractérisée en ce que** ladite au moins une surface latérale non profilée (38) est de courbure convexe en s'éloignant du corps d'aiguille (16).

8. Aiguille selon la revendication 6 ou la revendication 7, **caractérisée en ce que** sont prévues deux surfaces latérales (38) non profilées ou sensiblement non profilées.

9. Aiguille selon la revendication 8, **caractérisée en ce que** les deux surfaces latérales (38) non profilées ou sensiblement non profilées ont des orientations respectives pointant dans des directions opposées.

10. Aiguille selon l'une des revendications 6 à 8, **caractérisée en ce que** le corps d'aiguille (16) présente deux surfaces latérales à profil structuré (32), et **en ce que** sont prévues deux surfaces latérales (38) non profilées ou sensiblement non profilées reliant mutuellement les surfaces latérales à profil structuré (32).

11. Aiguille selon l'une des revendications précédentes, **caractérisée en ce que** le corps d'aiguille (16) est cintré.

12. Aiguille selon l'une des revendications précédentes, **caractérisée en ce que** ladite au moins une surface latérale à profil structuré (32) définit une surface extérieure, et en ce le grand nombre d'empreintes en creux (26) s'étendent à partir de la surface extérieure vers l'intérieur du corps d'aiguille (16).

13. Aiguille selon la revendication 12, **caractérisée en ce qu'**une profondeur des empreintes en creux (26) par rapport à la surface extérieure de ladite au moins une surface latérale à profil structuré (32), diminue, en partant d'une extrémité proximale de ladite au moins une zone de surface latérale à profil structuré (24), en direction la pointe d'aiguille (52).

14. Aiguille selon l'une des revendications précédentes, **caractérisée en ce que** le grand nombre d'empreintes en creux (26) présentent une configuration totalement ou partiellement en forme de pyramide.

15. Aiguille selon la revendication 14, **caractérisée en ce que** le grand nombre d'empreintes en creux (26) sont configurées totalement ou partiellement en forme de pyramides carrées (26).

16. Aiguille selon l'une des revendications précédentes, **caractérisée en ce que** le grand nombre d'empreintes en creux (26) sont réalisées par pressage.

17. Aiguille selon l'une des revendications 1 à 16, **caractérisée en ce que** le corps d'aiguille (16) est fabriqué par moulage par injection.

18. Aiguille selon la revendication 17, **caractérisée en ce que** le corps d'aiguille (16) est fabriqué par moulage par injection de métal (MIM - Metal Injection, Molding), par moulage par injection de poudre (PIM - Powder Injection Molding) ou par moulage par injection de matière plastique.

19. Aiguille selon l'une des revendications précédentes, **caractérisée en ce que** l'aiguille (54) est fabriquée en un métal, une céramique ou en matière plastique.

20. Aiguille selon l'une des revendications précédentes, **caractérisée en ce que** la pointe d'aiguille (50) est fabriquée par polissage électrochimique.

21. Aiguille selon l'une des revendications précédentes, **caractérisée en ce que** l'aiguille (54) présente à une extrémité proximale, au moins un chas, un trou oblong ou une entaille.

22. Aiguille selon la revendication 21, **caractérisée en ce que** le chas est réalisé sous la forme d'un évidement de passage de section transversale de forme circulaire, de forme ovale ou en forme de trou oblong, dans le corps d'aiguille (16).

23. Aiguille selon l'une des revendications précédentes, **caractérisée en ce que** la pointe d'aiguille (50) et/ou le corps d'aiguille en totalité, sont revêtus complètement ou partiellement d'une couche de silicone.

24. Procédé de fabrication d'une aiguille chirurgicale (54) présentant un corps d'aiguille allongé et une pointe d'aiguille (50, 52) formée à une extrémité distale (34) du corps d'aiguille (16), procédé selon lequel le corps d'aiguille (16) peut notamment être fabriqué à partir d'une ébauche brute cylindrique (10) allongée, selon lequel on munit le corps d'aiguille (16) d'au moins une surface latérale à profil structuré (32), et selon lequel, pour former au moins une zone de surface latérale à profil structuré (24), on munit ladite moins une surface latérale à profil structuré (32) d'un profil structuré, de façon telle que ladite au moins une zone de surface latérale à profil structuré (24) s'étende jusqu'à la pointe d'aiguille (50, 52),
**caractérisé en ce que** l'on réalise le profil structuré sous la forme d'un profil obtenu par estampage, qui présente un grand nombre d'empreintes en creux (26) obtenues par estampage.

25. Procédé selon la revendication 24, **caractérisé en ce que** l'on réalise ladite au moins une zone de surface latérale à profil structuré (24) de manière à ce qu'elle forme au moins la moitié de ladite au moins une surface latérale à profil structuré (32).

26. Procédé selon l'une des revendications 24 ou 25, **caractérisé en ce que** l'on munit le corps d'aiguille (16) de deux surfaces latérales à profil structuré (32), de manière à ce que les deux surfaces latérales à profil structuré (32) aient des orientations respectives pointant dans des directions opposées.

27. Procédé selon l'une des revendications 24 à 26, **caractérisé en ce que** l'on réalise le corps d'aiguille (16) de manière à ce qu'il présente, en section transversale, une forme rectangulaire ou carrée, ou sensiblement rectangulaire ou carrée.

28. Procédé selon l'une des revendications 24 à 27, **caractérisé en ce que** l'on réalise le corps d'aiguille (16) de manière à ce qu'il présente quatre surfaces latérales planes ou sensiblement planes (32, 38), et que l'une au moins des surfaces latérales (32) soit une surface latérale à profil structuré (32).

29. Procédé selon l'une des revendications 24 à 28, **caractérisé en ce que** l'on réalise le corps d'aiguille (16) de manière à ce qu'il présente au moins une surface latérale non profilée (38) adjacente à ladite au moins une surface latérale à profil structuré (32).

30. Procédé selon la revendication 29, **caractérisé en ce que** l'on réalise ladite au moins une surface latérale non profilée (38) de manière à être de courbure convexe en s'éloignant du corps d'aiguille (16).

31. Procédé selon la revendication 29 ou la revendication 30, **caractérisé en ce que** l'on réalise deux surfaces latérales (38) non profilées ou sensiblement non profilées.

32. Procédé selon la revendication 31, **caractérisé en ce que** l'on réalise les deux surfaces latérales (38) non profilées ou sensiblement non profilées de manière à ce que leurs orientations respectives pointent dans des directions opposées.

33. Procédé selon l'une des revendications 29 à 32, **caractérisé en ce que** l'on réalise le corps d'aiguille (16) de manière à ce qu'il présente deux surfaces latérales à profil structuré (32), et **en ce que** l'on réalise deux surfaces latérales (38) non profilées ou sensiblement non profilées reliant mutuellement les surfaces latérales à profil structuré (32).

34. Procédé selon l'une des revendications 24 à 33, **caractérisé en ce que** l'on cintre le corps d'aiguille (16) après la réalisation de ladite au moins une zone de surface latérale à profil structuré (24).

35. Procédé selon l'une des revendications 24 à 34, **caractérisé en ce que** ladite au moins une surface latérale à profil structuré (32) définit une surface extérieure, et en ce l'on forme le grand nombre d'empreintes en creux (26) vers l'intérieur du corps d'aiguille (16) à partir de la surface extérieure.

36. Procédé selon la revendication 35, **caractérisé en ce que** l'on réalise une profondeur des empreintes en creux (26) par rapport à la surface extérieure de ladite au moins une surface latérale à profil structuré (32), de façon à ce que cette profondeur diminue, en partant d'une extrémité proximale de ladite au moins une zone de surface latérale à profil structuré (24), en direction la pointe d'aiguille (50, 52).

37. Procédé selon l'une des revendications 24 à 36, **caractérisé en ce que** l'on réalise le grand nombre d'empreintes en creux (26) de manière à ce qu'elles présentent une configuration totalement ou partiellement en forme de pyramide.

38. Procédé selon la revendication 37, **caractérisé en ce que** l'on réalise le grand nombre d'empreintes en creux (26) de manière à ce qu'elles soient configurées totalement ou partiellement en forme de pyramides carrées (26).

39. Procédé selon l'une des revendications 24 à 38, **caractérisé en ce que** l'on réalise le grand nombre d'empreintes en creux (26) par pressage.

40. Procédé selon l'une des revendications 24 à 38, **caractérisé en ce que** l'on fabrique le corps d'aiguille (16) par moulage par injection.

41. Procédé selon la revendication 40, **caractérisé en ce que** l'on fabrique le corps d'aiguille (16) par moulage par injection de métal (MIM - Metal Injection, Molding), par moulage par injection de poudre (PIM - Powder Injection Molding) ou par moulage par injection de matière plastique.

42. Procédé selon l'une des revendications 24 à 41, **caractérisé en ce que** l'on fabrique l'aguille (54) en un métal, en matière plastique ou sous la forme d'une céramique par frittage.

43. Procédé selon l'une des revendications 24 à 42, **caractérisé en ce que** l'on fabrique la pointe d'aiguille (50) par polissage électrochimique.

44. Procédé selon l'une des revendications 24 à 43, **caractérisé en ce que** l'on munit l'aiguille (54), à une extrémité proximale, d'au moins un chas, un trou oblong ou une entaille.

45. Procédé selon la revendication 44, **caractérisé en ce que** l'on réalise le chas sous la forme d'un évidement de passage de section transversale de forme circulaire, de forme ovale ou en forme de trou oblong, dans le corps d'aiguille (16).

46. Procédé selon l'une des revendications 24 à 45, **caractérisé en ce que** l'on revêt complètement ou partiellement d'une couche de silicone, la pointe d'aiguille (50, 52) et/ou le corps d'aiguille (16) en totalité.
